# EUROPEAN PATENT APPLICATION

(11) **EP 4 285 857 A1**
(43) Date of publication of application: **06.12.2023**
(21) Application number: 21922467.2
(22) Date of filing: 18.11.2021
(51) Int. Cl.: A61B 34/30, B25J 9/00, B25J 13/00

(54) **VOICE PROMPT CONTROL METHOD AND SYSTEM FOR LAPAROSCOPIC SURGERY ROBOT**

(30) Priority: 28.01.2021 CN 202110115367
(71) Applicant: Harbin Intelligent Surgery Equipment Co., Ltd., Harbin, Heilongjiang 150000 (CN)
(72) Inventor: ZHAN, Mengxue, Harbin, Heilongjiang 150000 (CN); PANG, Haifeng, Harbin, Heilongjiang 150000 (CN)
(74) Representative: Zaboliene, Reda
(86) International application number: PCT/CN2021/131485
(87) International publication number: WO 2022/160877

(57) **Abstract**

The invention provides a voice prompt control method and system for a laparoscopic surgery robot. The voice prompt control method for the laparoscopic surgery robot comprises: acquiring real-time spatial positions of a plurality of calibration points on each robotic arm; obtaining the distances between corresponding positions of all the calibration points on any two robotic arms according to the real-time spatial positions of all the calibration points; obtaining the minimum distance between any two robotic arms according to the distances between the corresponding positions of all the calibration points on any two robotic arms; and determining, according to the minimum distance between any two robotic arms, whether to send out a voice broadcast. The surgery efficiency and the surgery success rate are improved.

## Description

### Technical Field

The invention relates to the technical field of medical equipment, and in particular to a voice prompt control method and system for a laparoscopic surgery robot.

### Background Art

With the development of laparoscopic technologies and surgery robots, the application range of laparoscopic surgery robots has been expanding. The laparoscopic surgery robots can partially replace boring, repetitive, and laborious operations. The use of robot system image stability and fine instruments can complete relatively difficult operations in traditional laparoscopic surgeries such as small tube anastomosis. The cooperation between doctors and robots can improve the surgery quality to a certain extent.

However, in some highly difficult surgeries, doctors and assistants need to focus on the end posture of an instrument in an abdominal cavity, and often ignore the movement positions of all joints of robotic arms and the relative positions of left and right main manipulators. Once the joints of the robotic arms reach extreme positions or there is a collision between the robotic arms, it will be impossible to control the robotic arms to reach a lesion point according to the original planning path of doctors, and the postures of the robotic arms need to be readjusted, or there is a collision between the two main manipulators, which will seriously affect the efficiency and success rate of the whole surgery.

### Summary of the Invention

The invention is aimed at improving the efficiency and success rate of laparoscopic surgeries.

In order to solve the above problem, the invention provides a voice prompt control method for a laparoscopic surgery robot, which includes:
acquiring real-time spatial positions of a plurality of calibration points on each robotic arm;
obtaining the distances between all corresponding positions of all the calibration points on any two robotic arms according to the real-time spatial positions of all the calibration points;
obtaining the minimum distance between any two robotic arms according to the distances between all the corresponding positions of all the calibration points on any two robotic arms; and
determining, according to the minimum distance between any two robotic arms, whether to send out a voice broadcast.

Optionally, the determining, according to the minimum distance between any two robotic arms, whether to send out a voice broadcast includes:
if the minimum distance between any two robotic arms, e.g., a first robotic arm and a second robotic arm, is less than or equal to a first set value, sending out a voice broadcast; and
if the maximum distance between the first robotic arm and the second robotic arm is greater than the first set value, skipping sending out a voice broadcast.

Optionally, the voice prompt control method for a laparoscopic surgery robot further includes:
acquiring the position information of all joints in the robotic arms; and
determining, according to the position information of a specified joint and the upper and lower limit values of the specified joint, whether to send out the voice prompt of the specified joint.

Optionally, the joints are angular rotation joints or linear displacement joints.

Optionally, the determining, according to the position information of the joint and the upper and lower limit values of the corresponding joint, whether to send out the voice prompt of the corresponding joint further includes:
if the absolute value of the difference value between the position information of the specified joint and the lower limit value in the upper and lower limit values is less than or equal to a second set value, sending out a voice broadcast; and
if the absolute value of the difference value between the position information of the specified joint and the lower limit value in the upper and lower limit values is greater than the second set value, sending out a voice broadcast.

Optionally, the determining, according to the position information of the joint and the upper and lower limit values of the corresponding joint, whether to send out the voice prompt of the corresponding joint further includes:
if the absolute value of the difference value between the position information of the specified joint and the upper limit value in the upper and lower limit values is less than or equal to the second set value, sending out a voice broadcast; and
if the absolute value of the difference value between the position information of the specified joint and the upper limit value in the upper and lower limit values is greater than the second set value, skipping sending out a voice broadcast.

Optionally, the acquiring the position information of all joints in the robotic arms includes: acquiring the position information of the corresponding joint through motor coding values at the joint.

Optionally, the voice prompt control method for a laparoscopic surgery robot further includes:
acquiring a first distance from a left manipulator to a right manipulator through a first infrared sensor on the left manipulator;
acquiring a second distance from the right manipulator to the left manipulator through a second infrared sensor on the right manipulator; and
determining whether to send out a voice broadcast according to the first distance and/or the second distance.

Optionally, the determining whether to send out a voice broadcast according to the first distance and/or the second distance includes:
if the first distance is less than or equal to a third set value, sending out a voice broadcast; and
if the first distance is greater than the third set value, determining the size of the second distance and the third set value; and if the second distance is less than or equal to the third set value, sending out a voice broadcast, and if the second distance is greater than the third set value, skipping out sending out a voice broadcast.

In addition, the invention further provides a voice prompt control system for a laparoscopic surgery robot, which includes:
an acquiring unit, configured to acquire real-time spatial positions of a plurality of calibration points on each robotic arm; and
a control unit, configured to obtain the distances between corresponding positions on any two robotic arms according to the real-time spatial positions of all the calibration points, further configured to obtain the minimum distance between any two robotic arms according to the distances between the corresponding positions on any two robotic arms, and further configured to determine, according to the minimum distance between any two robotic arms, whether to send out a voice broadcast.

Compared with the prior art, the voice prompt control method and system for the laparoscopic surgery robot provided by the invention have but are not limited to the following technical effects:
a plurality of calibration points are set at different positions on each robotic arm; real-time spatial positions of all the calibration points on each robotic arm are acquired; then the real-time spatial positions of two closest calibration points between any two robotic arms are obtained according to the real-time spatial positions of all the calibration points on all the robotic arms; the distance of the two closest calibration points between the two robotic arms is taken as the minimum distance between the two robotic arms; and according to the minimum distance between the two robotic arms, whether to send out a voice broadcast is determined, and whether to send out a prompt that the two robotic arms are about to collide is determined, thus ensuring that doctors and assistants need not worry about the collision between the robotic arms when they need to focus on the end posture of the instrument in the abdominal cavity, and further improving the efficiency and the success rate of the surgery.

### Brief Description of the Drawings

Fig.1 is a schematic flowchart I of a voice prompt control method for a laparoscopic surgery robot according to an embodiment of the invention.
Fig.2 is a schematic flowchart II of a voice prompt control method for a laparoscopic surgery robot according to an embodiment of the invention.
Fig.3 is a schematic flowchart III of a voice prompt control method for a laparoscopic surgery robot according to an embodiment of the invention.
Fig. 4 is a structural schematic diagram of a laparoscopic surgery robot with three robotic arms according to an embodiment of the invention.
Fig. 5 is a local schematic diagram of a robotic arm according to an embodiment of the invention.

### Detailed Description of the Invention

To make the above objects, features and advantages of the invention clearer and more comprehensible, specific embodiments of the invention will be described in detail below with reference to the accompanying drawings.

The terms "first", "second" and the like are only intended for description, but cannot be construed as indicating or implying relative importance or implicitly indicating the number of the specified technical features. Thus, the features defined with "first", "second" and the like may explicitly or implicitly include at least one of the features.

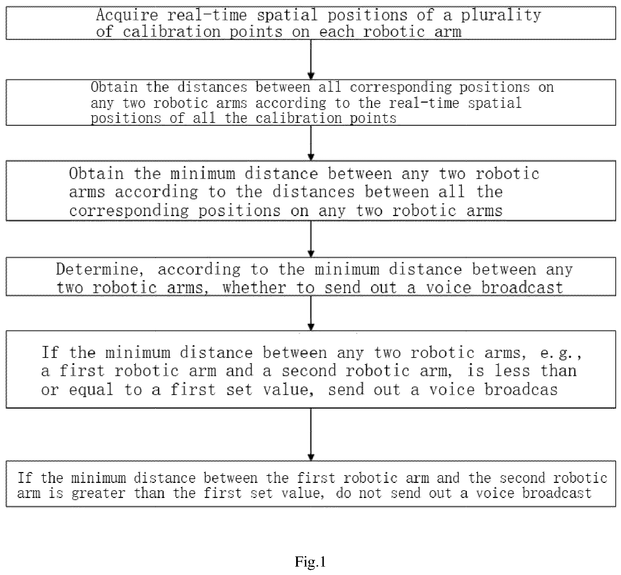

Referring to Fig. 1, the embodiment of the invention provides a voice prompt control method for a laparoscopic surgery robot, which includes:
acquiring real-time spatial positions of a plurality of calibration points on each robotic arm, wherein the ends of the robotic arms are suitable for installing an endoscope or a surgical instrument;
obtaining the distances between the corresponding positions of all the calibration points on any two robotic arms according to the real-time spatial positions of all the calibration points;
obtaining the minimum distance between any two robotic arms according to the distances between the corresponding positions of all the calibration points on any two robotic arms; and
determining, according to the minimum distance between any two robotic arms, whether to send out a voice broadcast.

Herein, a plurality of calibration points are set at different positions on each robotic arm; real-time spatial positions of all the calibration points on each robotic arm are acquired; then the real-time spatial positions of the two closest calibration points between any two robotic arms are obtained according to the real-time spatial positions of all the calibration points on all the robotic arms; the distance of the two closest calibration points between the two robotic arms is taken as the minimum distance between the two robotic arms; and according to the minimum distance between the two robotic arms, whether to send out a voice broadcast is determined, and whether to send out a prompt that the two robotic arms are about to collide is determined, thus ensuring that doctors and assistants need not worry about the collision between the robotic arms when they need to focus on the end posture of the instrument in the abdominal cavity, and further improving the operation efficiency and the success rate.

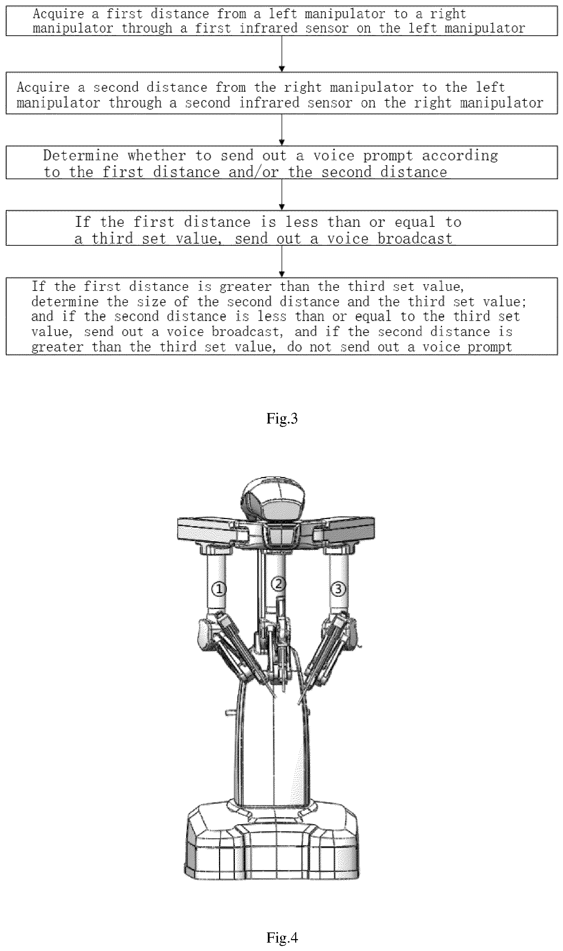

Herein, as shown in Fig. 4, a laparoscopic surgery robot with three robotic arms is taken as an example. The three robotic arms are respectively recorded as a first robotic arm (1), a second robotic arm (2), and a third robotic arm (3). For example, each robotic arm is provided with three calibration points. The three calibration points on the first robotic arm are respectively recorded as a first calibration point, a second calibration point, and a third calibration point. The three calibration points on the second robotic arm are respectively recorded as a fourth calibration point, a fifth calibration point, and a sixth calibration point. The three calibration points on the third robotic arm are respectively recorded as a seventh calibration point, an eighth calibration point, and a ninth calibration point. It can be understood that the position of the first calibration point on the first robotic arm is the same as that of the fourth calibration point on the second robotic arm, and the position of the fourth calibration point on the second robotic arm is the same as that of the seventh calibration point on the third robotic arm; the position of the second calibration point on the first robotic arm is the same as that of the fifth calibration point on the second robotic arm, and the position of the fifth calibration point on the second robotic arm is the same as that of the eighth calibration point on the third robotic arm; the position of the third calibration point on the first robotic arm is the same as that of the sixth calibration point on the second robotic arm, and the position of the sixth calibration point on the second robotic arm is the same as that of the ninth calibration point on the third robotic arm; the distance between the position of the first calibration point on the first robotic arm and the position of the fourth calibration point on the second robotic arm is recorded as Laa, the distance between the position of the first calibration point on the first robotic arm and the position of the fifth calibration point on the second robotic arm is recorded as Lab, and the distance between the position of the first calibration point on the first robotic arm and the position of the sixth calibration point on the second robotic arm is recorded as Lac; the distance between the position of the second calibration point on the first robotic arm and the position of the fourth calibration point on the second robotic arm is recorded as Lba, the distance between the position of the second calibration point on the first robotic arm and the position of the fifth calibration point on the second robotic arm is recorded as Lbb, and the distance between the position of the second calibration point on the first robotic arm and the position of the sixth calibration point on the second robotic arm is recorded as Lbc; the distance between the position of the third calibration point on the first robotic arm and the position of the fourth calibration point on the second robotic arm is recorded as Lca, the distance between the position of the third calibration point on the first robotic arm and the position of the fifth calibration point on the second robotic arm is Lcb, and the distance between the position of the third calibration point on the first robotic arm and the position of the sixth calibration point on the second robotic arm is recorded as Lcc; and thus, the minimum real-time distances between the first robotic arm and the second robotic arm are recorded as Laa, Lab, Lac, Lba, Lbb, Lbc, Lca, Lcb and Lcc.

It can be understood that optionally, each connecting rod in the robotic arms is provided with a calibration point, of course, the more and denser the number of the calibration points on the robotic arms is, the more accurate the minimum distance measured between the two robotic arms will be.

Referring to Fig. 1, optionally, the determining, according to the minimum distance between any two robotic arms, whether to send out a voice broadcast includes:
if the minimum distance between any two robotic arms, e.g. a first robotic arm and a second robotic arm, is less than or equal to a first set value, sending out a voice broadcast; and
if the minimum distance between the first robotic arm and the second robotic arm is greater than the first set value, skipping sending out a voice broadcast.

Herein, for example, if the minimum distance between the first robotic arm and the second robotic arm is less than or equal to the first set value of 5 cm, the voice broadcast is sent out. The content of the voice broadcast at least includes "Please note that the first robotic arm and the second robotic arm are about to collide", or similar contents are broadcast. If the minimum distance between the first robotic arm and the second robotic arm is greater than the first set value of 5 cm, the voice broadcast is not sent out.

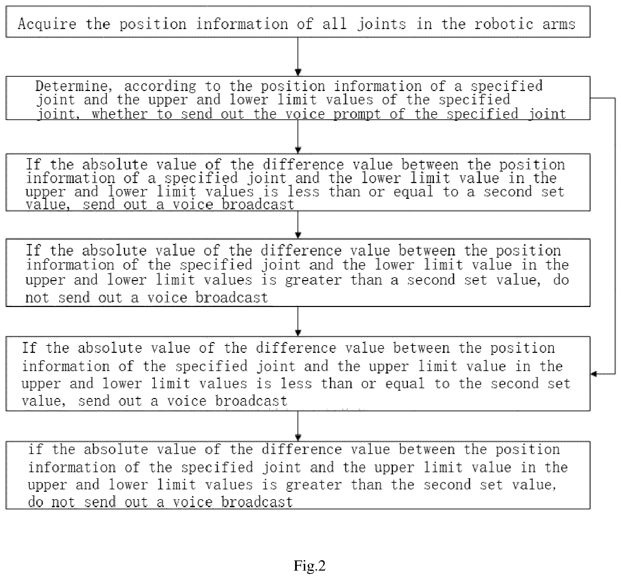

Referring to Fig. 2, optionally, the voice prompt control method for a laparoscopic surgery robot further includes:
acquiring the position information of all joints in the robotic arms; and
determining, according to the position information of a specified joint and the upper and lower limit values of the specified joint, whether to send out the voice prompt of the specified joint.

Herein, whether to send out a voice broadcast is determined by comparing the position information of all joints in the robotic arms with the upper and lower limit values corresponding to the specified joint, so that even if doctors and assistants concentrate all their attention on the end instrument, all joints will not move to a position corresponding to the upper limit value or a position corresponding to the lower limit value, and thus, the surgery efficiency and the surgery success rate are further improved.

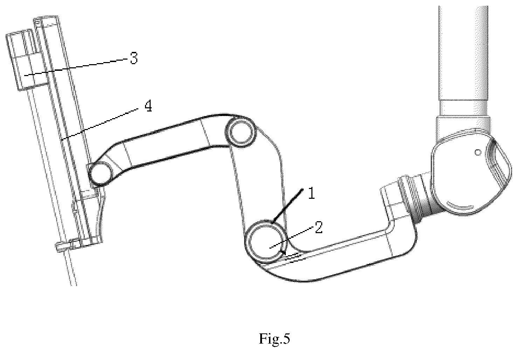

Referring to Fig. 5, optionally, the joint is an angular rotation joint or a linear displacement joint. For example, the specified rotation joint 1 can rotate around a rotating shaft 2, and the specified linear displacement joint 3 can move linearly along a sliding rail 4.

Optionally, the acquiring the position information of all joints in the robotic arms includes: acquiring the position information of the corresponding joints through motor coding values at the joints;
Herein, each joint is driven by a corresponding motor to move, then the rotation angle value of the joint can be obtained by acquiring a motor coding numerical value, and whether to send out a voice broadcast can be determined by comparing the rotation angle value of the joint with the upper and lower limit values of the joint.

Referring to Fig. 2, optionally, the determining, according to the position information of the joint and the upper and lower limit values of the corresponding joint, whether to send out the voice prompt of the corresponding joint includes:
if the absolute value of the difference value between the position information of a specified joint and the lower limit value in the upper and lower limit values is less than or equal to a second set value, sending out a voice broadcast; and
if the absolute value of the difference value between the position information of the specified joint and the lower limit value in the upper and lower limit values is greater than a second set value, skipping sending out a voice broadcast.

Herein, for example, the specified joint belongs to the first robotic arm, is an angular rotation joint, and is in the angular range of minus 40 degrees to 80 degrees, wherein minus 40 degrees is the lower limit value, and 80 degrees is the upper limit value. When the absolute value of the difference value between the position information of the specified joint and the lower limit value of minus 40 degrees is less than or equal to 5 degrees, a voice broadcast is sent out, and the content of the voice broadcast at least includes "the specified joint in the first robotic arm is about to reach the lower limit position", or similar contents are broadcast to prompt doctors and assistants. When the absolute value of the difference value between the position information of the specified joint and the lower limit value of minus 40 degrees is greater than 5 degrees, the voice broadcast is not sent out.

Referring to Fig. 2, optionally, the determining, according to the position information of the joint and the upper and lower limit values of the corresponding joint, whether to send out the voice prompt of the corresponding joint further includes:
if the absolute value of the difference value between the position information of the specified joint and the upper limit value in the upper and lower limit values is less than or equal to a second set value, sending out a voice broadcast; and
if the absolute value of the difference value between the position information of the specified joint and the upper limit value in the upper and lower limit values is greater than the second set value, skipping sending out a voice broadcast.

Herein, the specified joint which belongs to the first robotic arm, is an angular rotation joint, and is the angular range of minus 40 degrees to 80 degrees is still taken as an example, where minus 40 degrees is the lower limit value, and 80 degrees is the upper limit value. When the absolute value of the difference value between the position information of the specified joint and the upper limit value of 80 degrees is less than or equal to 5 degrees, a voice broadcast is sent out, and the content of the voice broadcast at least includes "the specified joint in the first robotic arm is about to reach the upper limit position", or similar contents are broadcast to prompt doctors and assistants. When the absolute value of the difference value between the position information of the specified joint and the lower limit value of minus 40 degrees is greater than 5 degrees, the voice broadcast is not sent out.

Referring to Fig. 3, optionally, the voice prompt control method for a laparoscopic surgery robot further includes:
acquiring a first distance from a left manipulator to a right manipulator through a first infrared sensor on the left manipulator;
acquiring a second distance from the right manipulator to the left manipulator through a second infrared sensor on the right manipulator; and
determining whether to send out a voice broadcast according to the first distance and/or the second distance.

Herein, the minimum distance between the left manipulator and the right manipulator is detected in real time by the two infrared sensors, which ensures that even if doctors and assistants concentrate all their attention on the end instrument, there will be no collision between the two main manipulators, and thus, the surgery efficiency and the surgery success rate are increased.

Referring to Fig. 3, optionally, the determining whether to send out a voice broadcast according to the first distance and/or the second distance includes:
if the first distance is less than or equal to a third set value, sending out a voice broadcast; and
if the first distance is greater than the third set value, determining the size of the second distance and the third set value; and if the second distance is less than or equal to the third set value, sending out a voice broadcast, and if the second distance is greater than the third set value, skipping sending out a voice broadcast.

Herein, as long as any one of the first distance and the second distance is less than or equal to the third set value of 3 cm, a voice broadcast will be sent out. The content of the voice broadcast at least includes "the left manipulator and the right manipulator are about to collide", or similar contents are broadcast to prompt doctors and assistants.

In addition, another embodiment of the invention further provides a voice prompt control system for a laparoscopic surgery robot, which includes:
an acquiring unit, configured to acquire real-time spatial positions of a plurality of calibration points on each robotic arm, and further configured to acquire the position information of all joints in the robotic arms, a first distance from a left manipulator to a right manipulator through a first infrared sensor on the left manipulator, and a second distance from the right manipulator to the left manipulator through a second infrared sensor on the right manipulator;
a control unit, configured to obtain the distances between corresponding positions on any two robotic arms according to the real-time spatial positions of all the calibration points, and further configured to obtain the minimum distance between any two robotic arms according to the distances between the corresponding positions on any two robotic arms, and determine whether to send out a voice broadcast according to the minimum distance between any two robotic arms, whether to send out voice prompts of the corresponding joints according to the position information of the joints and the upper and lower limit values of the corresponding joints, and whether to send out a voice broadcast according to the first distance and/the second distance.

The voice prompt control system for the laparoscopic surgery robot herein has the advantages of simple composition, low cost, high reliability, and good compatibility.

Although the invention is disclosed as above, the scope of protection of the invention is not limited thereto. Those skilled in the art can make various changes and modifications without departing from the spirit and scope of the invention, and all these changes and modifications shall fall within the scope claimed by the invention.

## Claims

1. A voice prompt control method for a laparoscopic surgery robot, **characterized by** comprising:
acquiring real-time spatial positions of a plurality of calibration points on each robotic arm;
obtaining the distances between all corresponding positions of all the calibration points on any two robotic arms according to the real-time spatial positions of all the calibration points;
obtaining the minimum distance between any two robotic arms according to the distances between all the corresponding positions of all the calibration points on any two robotic arms; and
determining, according to the minimum distance between any two robotic arms, whether to send out a voice broadcast.

2. The voice prompt control method for a laparoscopic surgery robot according to claim 1, **characterized in that** the determining, according to the minimum distance between any two robotic arms, whether to send out a voice broadcast comprises:
if the minimum distance between any two robotic arms, e.g., a first robotic arm and a second robotic arm, is less than or equal to a first set value, sending out a voice broadcast; and
if the maximum distance between the first robotic arm and the second robotic arm is greater than the first set value, skipping sending out a voice broadcast.

3. The voice prompt control method for a laparoscopic surgery robot according to claim 1, **characterized by** further comprising:
acquiring the position information of all joints in the robotic arms; and
determining, according to the position information of a specified joint and the upper and lower limit values of the specified joint, whether to send out the voice prompt of the specified joint.

4. The voice prompt control method for a laparoscopic surgery robot according to claim 3, **characterized in that** the joints are angular rotation joints or linear displacement joints.

5. The voice prompt control method for a laparoscopic surgery robot according to claim 3, **characterized in that** the determining, according to the position information of a specified joint and the upper and lower limit values of the specified joint, whether to send out the voice prompt of the specified joint comprises:
if the absolute value of the difference value between the position information of the specified joint and the lower limit value in the upper and lower limit values is less than or equal to a second set value, sending out a voice broadcast; and
if the absolute value of the difference value between the position information of the specified joint and the lower limit value in the upper and lower limit values is greater than the second set value, skipping sending out a voice broadcast.

6. The voice prompt control method for a laparoscopic surgery robot according to claim 5, **characterized in that** the determining, according to the position information of the joint and the upper and lower limit values of the corresponding joint, whether to send out the voice prompt of the corresponding joint further comprises:
if the absolute value of the difference value between the position information of the specified joint and the upper limit value in the upper and lower limit values is less than or equal to the second set value, sending out a voice broadcast; and
if the absolute value of the difference value between the position information of the specified joint and the upper limit value in the upper and lower limit values is greater than the second set value, skipping sending out a voice broadcast.

7. The voice prompt control method for a laparoscopic surgery robot according to claim 3, **characterized in that** the acquiring the position information of all joints in the robotic arms comprises:
acquiring the position information of the corresponding joint through motor coding values at the joint.

8. The voice prompt control method for a laparoscopic surgery robot according to any one of claims 1 to 7, **characterized by** further comprising:
acquiring a first distance from a left manipulator to a right manipulator through a first infrared sensor on the left manipulator;
acquiring a second distance from the right manipulator to the left manipulator through a second infrared sensor on the right manipulator; and
determining whether to send out a voice broadcast according to the first distance and/or the second distance.

9. The voice prompt control method for a laparoscopic surgery robot according to claim 8, **characterized in that** the determining whether to send out a voice broadcast according to the first distance and/or the second distance comprises:
if the first distance is less than or equal to a third set value, sending out a voice broadcast; and
if the first distance is greater than the third set value, determining the size of the second distance and the third set value; and if the second distance is less than or equal to the third set value, sending out a voice broadcast, and if the second distance is greater than the third set value, skipping sending out a voice broadcast.

10. A voice prompt control system for a laparoscopic surgery robot, **characterized by** comprising:
an acquiring unit, configured to acquire real-time spatial positions of a plurality of calibration points on each robotic arm; and
a control unit, configured to obtain the distances between corresponding positions on any two robotic arms according to the real-time spatial positions of all the calibration points, further configured to obtain the minimum distance between any two robotic arms according to the distances between the corresponding positions on any two robotic arms, and further configured to determine, according to the minimum distance between any two robotic arms, whether to send out a voice broadcast.
